Europäisches Patentamt

European Patent Office

Office européen des brevets

(19)

(11) Publication number: **0 204 326 B1**

## EUROPEAN PATENT SPECIFICATION

(12)

(45) Date of publication of patent specification: **25.03.92**  (51) Int. Cl.⁵: **C12N 15/31**, C12P 13/06, C12P 13/08, C07H 21/04

(21) Application number: **86107595.0**

(22) Date of filing: **04.06.86**

(54) **Process for producing L-threonine and L-isoleucine.**

(30) Priority: **05.06.85 JP 121674/85**
**31.01.86 JP 19801/86**
**02.04.86 JP 76298/86**

(43) Date of publication of application:
**10.12.86 Bulletin 86/50**

(45) Publication of the grant of the patent:
**25.03.92 Bulletin 92/13**

(84) Designated Contracting States:
**DE FR GB**

(56) References cited:
**EP-A- 0 088 166**
**EP-A- 0 131 171**
**EP-A- 0 137 348**
**EP-A- 0 179 338**

(73) Proprietor: **KYOWA HAKKO KOGYO CO., LTD.**
**6-1, Ohtemachi 1-chome**
**Chiyoda-ku Tokyo-to(JP)**

(72) Inventor: **Katsumata, Ryoichi**
**Popuraoka Coopu 6-401 2-12-3, Naruse**
**Machida-shi Tokyo(JP)**
Inventor: **Mizukami, Toru**
**1-6-16, Asahi-machi**
**Machida-shi Tokyo(JP)**
Inventor: **Yokoi, Masako**
**Popuraoka Coopu 21-205 2-32-4, Narusedai**
**Machida-shi Tokyo(JP)**
Inventor: **Kikuchi, Yasuhiro**
**3-9-9, Naka-machi**
**Machida-shi Tokyo(JP)**
Inventor: **Oka, Tetsuo**
**2360-17, Nara-machi Midori-ku**
**Yokohama-shi Kanagawa-ken(JP)**

(74) Representative: **Vossius & Partner**
**Siebertstrasse 4 P.O. Box 86 07 67**
**W-8000 München 86(DE)**

**EP 0 204 326 B1**

## Description

As for the processes for producing L-threonine and L-isoleucine by fermentation using microorganisms belonging to the genus Corynebacterium or Brevibacterium, processes using mutants derived from wild-type strains of the said bacteria are known. As the mutant strains producing L-threonine and L-isoleucine, strains having an amino acid-requiring mutation and/or an amino acid analog-resistant mutation are known, as disclosed, for example, in Japanese Unexamined Published Patent Application No. 19087/72 or Japanese Patent Publication No. 32070/79, etc. Besides the processes using the mutant strains, processes using strains established by a recombinant DNA technique are known. For example, there are disclosed processes for producing L-threonine or L-isoleucine by fermentation using a strain belonging to the genus Corynebacterium or Brevibacterium and carrying a recombinant plasmid DNA which contains a DNA fragment carrying the genetic information of an enzyme responsible for the threonine biosynthesis in Escherichia coli (Japanese Unexamined Published Patent Application Nos. 126789/83 and 30693/85).

Furthermore, there is disclosed a process for producing L-threonine or L-isoleucine by the strains belonging to the genus Corynebacterium or Brevibacterium and carrying a recombinant plasmid DNA which contains a gene coding for homoserine dehydrogenase (referrred to as HD hereinafter) of the genus Brevibacterium (referred to as HD gene hereinafter) (Japanese Unexamined Published Patent Application No. 12995/85).

With the recent increase in the demand for L-threonine and L-isoleucine, an improvement in the processes for producing these amino acids has been desired. To meet this problem, the present inventors have made extensive studies for increasing the ability of the strains of the genus Corynebacterium or Brevibacterium to produce L-threonine and L-isoleucine by a recombinant DNA technique.

The present inventors nave found that the ability of a strain of the genus Corynebacterium or Brevibacterium to produce L-threonine and L-isoleucine to be biosynthesized from L-threonine as a precursor can be considerably improved by introducing into the strain a recombinant plasmid DNA containing genetic information of HD and homoserine kinase (referred to as HK hereinafter) at the same time, among the enzymes responsible for the biosynthesis of L-threonine in a microorganism belonging to the genus Corynebacterium or Brevibacterium and have established the present invention. As for L-threonine-producing strains or the L-isoleucine-producing strains carrying a recombinant plasmid DNA containing a gene originating from a strain of the genus Corynebacterium or Brevibacterium, a strain containing HD gene has been disclosed, as mentioned before, but there have been no examples of strains containing both genes, HD gene and a gene coding for HK (referred to as HK gene hereinafter), and it has been found in the present invention for the first time that the recombinant DNA containing both genes can remarkably contribute to the ability to produce L-threonine and L-isoleucine.

The present invention relates to a process for producing L-threonine or L-isoleucine, which comprises constructing a recombinant DNA containing genes coding for both enzymes of HD and HK responsible for the threonine biosynthesis in a microorganism belonging to the genus Corynebacterium or Brevibacterium, incorporating the recombinant DNA in a microorganism belonging to the genus Corynebacterium or Brevibacterium, culturing the microorganism in a medium, and recovering L-threonine or L-isoleucine accumulated in the culture broth. Thus, the present invention relates to the bioindustrial field, and particularly to the field of producing L-threonine and L-isoleucine useful in the industries of medicine, food, and animal feed.

Fig. 1 shows a cleavage map of pChom1 for restriction enzymes SalI, PstI, EcoRI and BamHI and the steps for constructing pChom1. The molecular size of plasmids is given in kilobases (Kb). Both HD and HK genes are contained in the chromosomal DNA fragment of pChom1 indicated by the heavy line.

Fig. 2 shows a cleavage map of the SalI fragment of 3.6 Kb cloned on pChom1 for typical restriction enzymes.

According to the present invention, L-threonine and L-isoleucine can be produced in high yield by culturing in a medium a microorganism belonging to the genus Corynebacterium or Brevibacterium and containing a recombinant DNA that contains both HD and HK genes of a microorganism belonging to the genus Corynebacterium or Brevibacterium and a vector DNA, accumulating L-threonine or L-isoleucine in the culture broth, and recovering L-threonine or L-isoleucine therefrom. As for the strain of the genus Corynebacterium or Brevibacterium to be used as a host microorganism, all the microorganisms known as the coryneform glutamic acid-producing bacteria can be used, and preferably the following strains can be used:

2

| Corynebacterium glutamicum | ATCC 31833 |
|---|---|
| Corynebacterium glutamicum | ATCC 13032 |
| Corynebacterium acetoacidophilum | ATCC 13870 |
| Corynebacterium herculis | ATCC 13868 |
| Corynebacterium lilium | ATCC 15990 |
| Brevibacterium divaricatum | ATCC 14020 |
| Brevibacterium flavum | ATCC 14067 |
| Brevibacterium immariophilium | ATCC 14068 |
| Brevibacterium lactofermentum | ATCC 13869 |
| Brevibacterium thiogenitalis | ATCC 19240 |

As the host microorganism, L-threonine- or L-isoleucine-non-producing strains can be used, but preferably L-threonine-, L-isoleucine- or L-lysine-producing strains are used. The L-threonine-, L-isoleucine- or L-lysine-producing strains can be constructed by deriving mutants which carry an amino acid-requiring mutation, an amino acid analog-resistant mutation, or a combination of these mutations (PRESCOTT AND DUNN'S INDUSTRIAL MICROBIOLOGY 4th ed. compiled by G. Reed, The AVI Publishing Company Inc. Conn. 1982, pp748 - 801, K. Nakayama).

In the present invention, any microorganism can be used as a supply source of both HD and HK genes, so long as it is a coryneform glutamic acid-producing microorganism having HD and HK activities. For example, wild-type strains belonging to the genus Corynebacterium or Brevibacterium, or L-lysine-, L-threonine- or L-isoleucine-producing mutant strains derived therefrom can be used. The chromosomal DNA of these strains can be isolated by subjecting the cells treated with penicillin during the culturing to lysis with lysozyme and a surfactant, removing proteins according to the ordinary procedure and precipitating the DNA with ethanol as disclosed in Japanese Unexamined Published Patent Application No. 126789/83 by the present inventors.

The vector for recombination of a DNA fragment containing both HD and HK genes from the chromosomal DNA is not particularly restricted, so long as it is autonomously replicable in a microorganism belonging to the genus Corynebacterium or Brevibacterium. For example, the plasmids developed by the present inventors, pCG1 (Japanese Unexamined Published Patent Application No. 134500/82), pCG2 (Japanese Unexamined Published Patent Application No. 35197/83), pCG4 and pCG11 (both in Japanese Unexamined Published Patent Application No. 183799/82), pCE54 and pCB101 (both in Japanese Unexamined Published Patent Application No. 105999/83), pCE51 (Japanese Unexamined Published Patent Application No. 34197/85), pCE52 and pCE53 [both in Molecular and General Genetics 196, 175 (1984)], etc. can be used. The plasmid vectors can be isolated and purified as CCC-DNA by subjecting cells to lysis with lysozyme and a surfactant, preparing cleared lysates, and precipitating the DNA with polyethyleneglycol, followed by cesium chloride-ethidium bromide density-gradient centrifugation, as disclosed by the present inventors in Japanese Unexamined Published Patent Application Nos. 134500/82 and 186489/82.

A recombinant DNA containing both HD and HK genes and a vector plasmid can be produced together with a mixture of various recombinants according to the ordinary procedures [Methods in Enzymology, 68 - (1979)], by cleaving the chromosomal DNA and the vector plasmid with a restriction enzyme, followed by, if necessary, treatment of the termini with terminal transferase or DNA polymerase, and by ligating both DNAs with a DNA ligase.

A recombinant plasmid containing both HD and HK genes can be obtained by transforming a homoserine (or methionine and threonine)-requiring, HD-deficient mutant strain or a threonine-requiring and homoserine-secreting, HK-deficient mutant strain, derived from a strain of the genus Corynebacterium or Brevibacterium, according to ordinary mutation procedures using the said mixture of recombinants, and by selecting a homoserine or threonine-non-requiring transformant, respectively. Transformation of a strain of the genus Corynebacterium or Brevibacterium can be carried out according to a method using protoplasts developed by the present inventors (Japanese Unexamined Published Patent Application Nos. 186492/82 and 186489/82, specifically refer to Examples). The recombinant plasmid containing both HD and HK genes is identified by the ability of complementing the deficiency in HD and HK by retransformation with the thus obtained recombinant plasmid DNA.

When the chromosomal DNA of a wild-type strain of the genus Corynebacterium or Brevibacterium is used as a supply source, a recombinant containing both HD and HK genes of wild type is obtained. The productivity of L-threonine and L-isoleucine can be improved by incorporating the recombinant plasmid into a strain of the genus Corynebacterium or Brevibacterium. It is known that in the strains of the genus Corynebacterium or Brevibacterium the HD responsible for the biosynthesis of threonine undergoes feedback inhibition by threonine, and controls the threonine synthesis [Agricultural Biological Chemistry 38 - (5), 993 (1974)], and thus the productivity of L-threonine and L-isoleucine can be further improved by using a recombinant plasmid containing a gene coding for the mutant HD relieved from inhibition by threonine.

The recombinant plasmid containing the mutant HD gene can be obtained in the same manner as in the case of the wild-type gene by isolating a mutant strain resistant to a threonine analog, e.g. $\alpha$-amino-$\beta$-hydroxyvaleric acid (referred to as AHV hereinafter), HD activity of which is relieved from the inhibition by threonine as described in Agricultural Biological Chemistry, 38 (5), 993 (1974), and by using its chromosomal DNA as a supply source. Alternatively, the recombinant plasmid containing the gene coding for HD freed from the inhibition by threonine can be obtained by subjecting a strain or the genus Corynebacterium or Brevibacterium carrying a recombinant plasmid that contains a wild-type HD gene to mutagenic treatment according to the ordinary procedure, thereby endowing the strain with a resistance to a threonine analog.

The recombinant plasmid containing both HD and HK genes of wild-type or mutant-type can be introduced into a microorganism of the genus Corynebacterium or Brevibacterium by said transformation using protoplasts. Production of L-threonine or L-isoleucine by the strains containing the recombinant plasmid is carried out according to a conventional culturing process for producing L-threonine or L-isoleucine by fermentation. That is, by culturing the transformant in an ordinary medium containing a carbon source, a nitrogen source, inorganic compounds, amino acids, vitamins, etc. under aerobic conditions while adjusting the temperature, pH, etc., L-threonine or L-isoleucine is accumulated in the medium during the culturing, and recovered therefrom.

As the carbon source, carbohydrates such as glucose, glycerol, fructose, sucrose, maltose, mannose, starch, starch hydrolyzate, molasses, etc.; polyalcohols; and various organic acids such as pyruvic acid, fumaric acid, lactic acid, acetic acid, etc. can be used. Furthermore, hydrocarbons, alcohols, etc. can be used depending upon the assimilability of the microorganism to be used. Particularly, cane molasses is preferably used.

As the nitrogen source, ammonia; various inorganic and organic ammonium salts such as ammonium chloride, ammonium sulfate, ammonium carbonate, ammonium acetate, etc.; urea and other nitrogen-containing materials as well as various nitrogen-containing organic materials such as peptone, NZ-amine, meat extract, yeast extract, corn steep liquor, casein hydrolyzate, fish meal or its digested product, chrysalis hydrolyzate, etc. can be used.

As the inorganic materials, dipotassium hydrogen phosphate, potassium dihydrogen phosphate, ammonium sulfate, ammonium chloride, magnesium sulfate, sodium chloride, terrous sulfate, manganese sulfate, calcium carbonate, etc. can be used. If the vitamins, the amino acids, etc. required for the growth of the microorganism can be supplied to the medium from other medium components described above, it is not necessary to add them to the medium.

Culturing is carried out under aerobic conditions, for example, by shaking culture or by aeration-stirring culture. Generally, preferable culturing temperature is 20 to 40°C. The pH of the medium is preferably maintained around neutrality. Usually, by culturing for 1 to 5 days, L-threonine and/or L-isoleucine is accumulated in the medium. After culturing, the cells are removed from the culture liquor, and then L-threonine and/or L-isoleucine is recovered from the culture liquor according to a known procedure, for example, by activated carbon treatment or by ion exchange resin treatment.

Thus, L-threonine and/or L-isoleucine can be produced in high yield by using a microorganism of the genus Corynebacterium or Brevibacterium containing a recombinant plasmid that contains both HD and HK genes of a microorganism belonging to the genus Corynebacterium or Brevibacterium.

An example of the present invention is given below:

Example

(1) Preparation of chromosomal DNA of Corynebacterium glutamicum ATCC 31833 and of vector pCE54:

A seed culture of Corynebacterium glutamicum ATCC 31833 grown in NB medium (a medium containing 20g of bouillon powder and 5g of yeast extract in 1ℓ of deionized water, and adjusted to pH 7.2) was inoculated into 400 mℓ of semi-synthetic medium SSM [a medium containing 20g of glucose, 10g of $(NH_4)_2SO_4$, 3g of urea, 1g of yeast extract, 1g of $KH_2PO_4$, 0.4g of $MgCl_2 \cdot 6H_2O$, 10 mg of $FeSO_4 \cdot 7H_2O$, 0.2 mg of $MnSO_4 \cdot 4\text{-}6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 $\mu$g of biotin and 1 mg of thiamine hydrochloride in 1ℓ of water, and adjusted to pH 7.2] and was subjected to shaking culture at 30°C. The optical density at 660 nm (OD) was determined with a Tokyo Koden colorimeter, and when the OD reached 0.2, penicillin G was added to a final concentration of 0.5 unit/mℓ. Culturing was continued until the OD reached 0.6.

Then, the cells were collected from the culture broth and washed with TES buffer solution [0.03M Tris-(hydroxymethyl) amino-methane (referred to as Tris hereinafter)-HCl, 0.005M EDTA (disodium ethylenediaminetetraacetate), and 0.05M NaCl, pH 8.0]. The washed cells were suspended in 10 mℓ of a lysozyme solution (25% sucrose, 0.1M NaCl, 0.05M Tris, and 0.8 mg/mℓ lysozyme, pH 8.0; the same solution is referred to hereinafter as lysozyme solution), and subjected to reaction at 37°C for 4 hours. High molecular chromosomal DNA was isolated from the collected cells according to the method of Saito, et al. [Saito, H. et al.: Biochim. Biophys. Acta, 72, 619 (1963)].

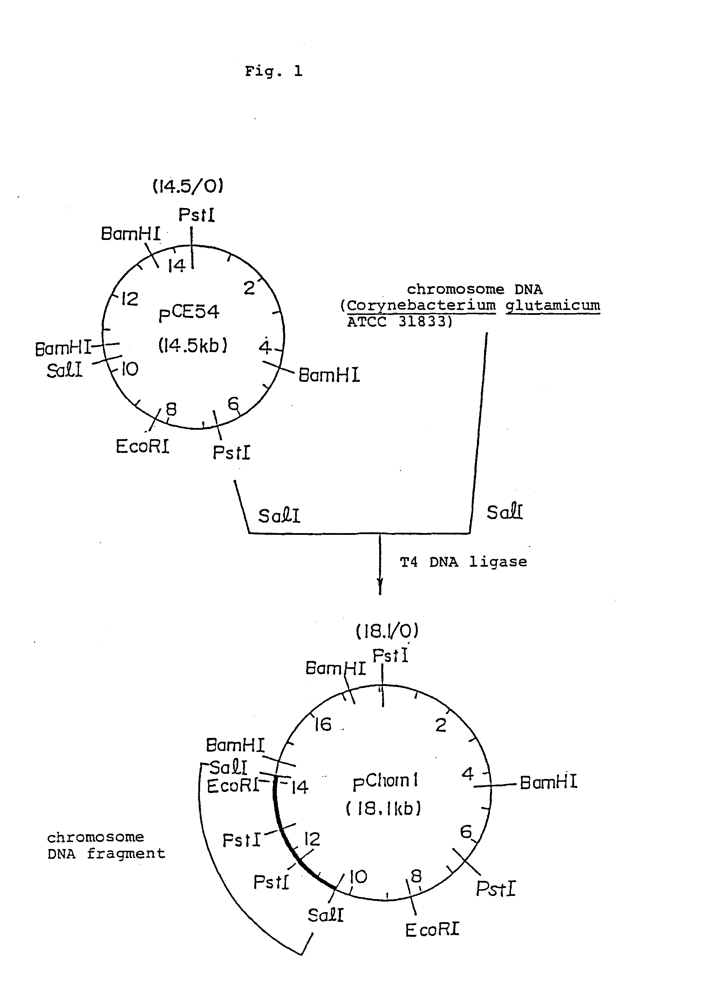

pCE54 (Japanese Unexamined Published Patent Application No. 105999/83) used as the vector is a plasmid prepared by combining plasmid pCG2 of Corynebacterium glutamicum (Japanese Unexamined Published Patent Application No. 35197/83 filed by the present inventors) with plasmid pGA22 of Escherichia coli [J. Bacteriol. 140, 400 (1979)]. More particularly, it is prepared by combining pCG2 with pGA22 at their single PstI cleavage sites (see Fig. 1). pCE54 was isolated from cultured cells of Corynebacterium glutamicum ATCC 39019 (a strain having a lysozyme-sensitive mutation and derived from Corynebacterium glutamicum ATCC 31833) carrying pCE54 in the following manner.

The cells were grown till the OD reached about 0.7 by shaking culture at 30°C in 400 mℓ of NB medium and were collected. After washing with TES buffer solution, the cells were suspended in 10 mℓ of a lysozyme solution and subjected to reaction at 37°C for 2 hours. Then, 2.4 mℓ of 5M NaCl, 0.6 mℓ of 0.5M EDTA (pH 8.5), and 4.4 mℓ of a solution containing 4% sodium laurylsulfate and 0.7M NaCl were successively added to the reaction solution, and the thus obtained mixture was gently mixed and placed on ice water for 15 hours. The lysate was transferred into a centrifuge tube and subjected to centrifugation at 69,400 x g at 4°C for 60 minutes to recover a supernatant. Then, polyethyleneglycol (PEG) 6,000 (Nakarai Kagaku Yakuhin Co.) in an amount corresponding to 10% by weight was added thereto, and the thus obtained mixture was gently mixed. The solution was placed on ice water, and after 10 hours, subjected to centrifugation at 1,500 x g for 10 minutes to recover pellets. Then, 5 mℓ of TES buffer solution was added to gently redissolve the pellets, and 2.0 mℓ of 1.5 mg/mℓ ethidium bromide was added thereto. Cesium chloride was added and gently dissolved to adjust the density of the solution to 1.580.

The solution was subjected to ultracentrifugation at 105,000 x g at 18°C for 48 hours, and a band at a high density level at a lower position of the centrifuge tube detected under ultraviolet irradiation was withdrawn at the side of the centrifuge tube with a syringe to separate the pCE54 plasmid DNA. The thus obtained fraction was treated 5 times with an equal volume of an isopropyl alcohol solution (consisting of 90% by volume isopropyl alcohol and 10% by volume TES buffer solution and containing cesium bromide at the saturated concentration) to remove ethidium bromide by extraction, and then dialyzed against TES buffer solution.

(2) Cloning of a DNA fragment containing HD and HK genes:

First, 6 units of SalI (Takara Shuzo Co.) was added to 60 µℓ of a reaction solution for restriction enzyme SalI (10 mM Tris-HCl, 6 mM MgCl₂ and 200 mM NaCl, pH 7.5) containing 3 µg of pCE54 plasmid DNA prepared above, and the mixture was subjected to reaction at 37°C for 60 minutes, and then heated at 65°C for 10 minutes to stop the reaction.

Separately, 4 units of SalI was added to 140 µℓ of the reaction solution for SalI containing 8 µg of chromosomal DNA of Corynebacterium glutamicum ATCC 31833, and the mixture was subjected to reaction at 37°C for 60 minutes, and then heated at 65°C for 10 minutes to stop the reaction.

Both reaction mixtures were mixed, and 40 µℓ of a 10-fold concentrated buffer solution for T4 ligase (660 mM Tris-HCl, 66 mM MgCl₂, and 100 mM dithiothreitol, pH 7.6), 40 µℓ of 5 mM ATP, 0.3 µℓ of T4 ligase (1 unit/µℓ, Takara Shuzo Co.) and 120 µℓ of purified water were added thereto. The mixture was subjected to reaction at 12°C for 16 hours.

The ligase reaction mixture was used to transform K53 strain derived from a lysozyme-sensitive mutant strain originating from Corynebacterium glutamicum ATCC 31833 [K53 strain carries a homoserine-requiring mutation (HD defect) and a leucine-requiring mutation]. strain K53 was deposited as FERM P-8257 with the Fermentation Research Institute (FRI), Agency of Industrial Science and Technology on May 23, 1985 and transferred to the deposit under the Budapest Treaty as FERM BP-1052 on May 19, 1986.

For the transformation, protoplasts prepared in the following manner were used.

A seed culture of strain K53 was inoculated in NB medium and subjected to shaking culture at 30°C, and the cells were collected when the OD reached 0.6. The cells were suspended in a solution (pH 7.6) containing 1 mg/mℓ lysozyme in RCGP medium [5g of glucose, 5g of Casamino acid, 2.5g of yeast extract,

3.5g of $K_2HPO_4$, 1.5g of $KH_2PO_4$, 0.41g of $MgCl_2 \cdot 6H_2O$, 10 mg of $FeSO_4 \cdot 7H_2O$, 2 mg of $MnSO_4 \cdot 4\text{-}6H_2O$, 0.9 mg of $ZnSO_4 \cdot 7H_2O$, 0.04 mg of $(NH_4)_6Mo_7O_{24} \cdot 4H_2O$, 30 $\mu$g of biotin, 2 mg of thiamine hydrochloride, 135g of disodium succinate, and 30g of polyvinylpyrrolidone (molecular weight: 10,000) in 1$\ell$ of water] to yield about $10^9$ cells/m$\ell$, and the suspension was transferred into an L-tube and subjected to gentle shaking culture at 30°C for 5 hours to yield protoplasts.

Then, 0.5 m$\ell$ of the protoplast suspension was transferred into a small test tube, and subjected to centrifugation at 2,500 x g for 5 minutes. The protoplasts were suspended again in 1 m$\ell$ of TSMC buffer solution (10 mM $MgCl_2$, 30 mM $CaCl_2$, 50 mM Tris-HCl, and 400 mM sucrose, pH 7.5), subjected to centrifugation, and then suspended in 0.1 m$\ell$ of TSMC buffer solution. Then, 100 $\mu\ell$ of a 1:1 mixture of TSMC buffer solution at a two-fold concentration and the said ligase reaction solution was added to the cell suspension, and 0.8 m$\ell$ of TSMC buffer solution containing 20% PEG 6,000 was added thereto. After 3 minutes, 2 m$\ell$ of RCGP medium (pH 7.2) was added thereto, and the mixture was subjected to centrifugation at 2,500 x g for 5 minutes to remove the supernatant. The pelleted protoplasts were suspended in 1 m$\ell$ of RCGP medium, and then 0.2 m$\ell$ of the suspension was spread onto RCGP agar medium (a medium containing 1.4% agar in RCGP medium, pH 7.2) containing 300 $\mu$g/m$\ell$ kanamycin, and cultured at 30°C for 7 days.

Colonies grown on the agar medium were collected. The cells were centrifuged, washed twice with physiological saline solution, and then suspended in 1 m$\ell$ of physiological saline solution. Then, the cell suspension was spread on to minimal agar medium M1 [a medium containing 10g of glucose, 1g of $NH_4H_2PO_4$, 0.2g of KCl, 0.2g of $MgSO_4 \cdot 7H_2O$, 10 mg of $FeSO_4 \cdot 7H_2O$, 0.2 mg of $MnSO_4 \cdot 4\text{-}6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 50 $\mu$g of biotin, 2.5 mg of p-aminobenzoic acid, 1 mg of thiamine hydrochloride, and 16g of agar in 1$\ell$, and adjusted to pH 7.2] containing 50 $\mu$g/m$\ell$ leucine and 20 $\mu$g/m$\ell$ kanamycin, and cultured at 30°C for 3 days, and transformants having a homoserine non-requirement and a kanamycin resistance were selected.

The transformants were cultured in NB medium, and plasmid DNAs were isolated from the cells by the same method as used for isolating pCE54 in said step (1).

It was found as a result of analysis by digestion with various restriction enzymes and agarose gel electrophoresis that the plasmid obtained from one of the transformants and named "pChom1" was a plasmid wherein a SalI DNA fragment of 3.6 Kb was inserted at the single SalI cleavage site of pCE54. The SalI DNA fragment had two PstI cleavage sites and one EcoRI cleavage site at the positions shown in Fig. 1.

The transformant of strain K53 selected by kanamycin resistance after retransformation with pChom1 DNA in the same manner as above showed homoserine non-requirement at the same time, and the plasmid isolated therefrom had the same structure as that of pChom1. These results support that the HD gene of Corynebacterium glutamicum ATCC 31833 was cloned in pChom1.

The presence of HK gene in pChom1 was confirmed in the following manner.

Protoplasts of threonine-requiring, homoserine-producing, HK-deficient mutant strain K54 derived from Corynebacterium glutamicum ATCC 31833 and deposited as FERM P-8258 with the FRI on May 23, 1985 (transferred to the deposit under the Budapest Treaty as FERM BP-1053 on May 19, 1986) were transformed with pChom1 DNA.

Protoplasts of the strain were prepared from penicillin-treated cells in the following manner.

First, 0.1 m$\ell$ of a seed culture obtained using NB medium was inoculated in 10 m$\ell$ of SSM medium containing 100 $\mu$g/m$\ell$ threonine, and subjected to shaking culture at 30°C. Penicillin G was added to a final concentration of 0.45 unit/m$\ell$ when the OD reached 0.15. Culturing was further continued, and when the OD reached 0.6, the cells were collected and treated with lysozyme by the same method as used above for preparing the protoplasts of strain K53 to produce a protoplast. Transformation was also conducted in the same manner as above, and a transformant was selected on RCGP agar medium containing 300 $\mu$g/m$\ell$ kanamycin. The kanamycin-resistant transformant was threonine non-requiring at the same time.

The transformant was subjected to shaking culture in 400 m$\ell$ of SSM medium, and penicillin G was added to 0.5 unit/m$\ell$ when the OD reached 0.2. Culturing was further continued till the OD reached about 0.6, and the cells were collected. The collected cells were subjected to lysis in the same manner as described in step (1), and a plasmid was isolated from the lysate by cesium chloride-ethidium bromide density gradient centrifugation. As a result of analysis by agarose gel electrophoresis after digestion with various restriction enzymes, it was confirmed that the plasmid was the same as pChom1.

It was found from the foregoing that the SalI DNA fragment of 3.6 Kb cloned in pChom1 had both HD and HK genes.

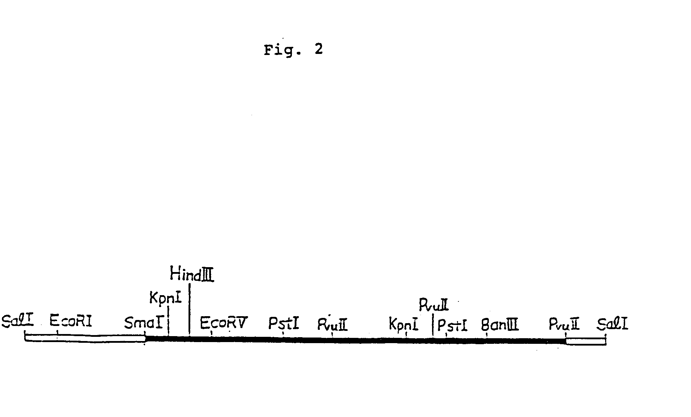

A cleavage map of the SalI fragment of 3.6 Kb cloned in pChom1 for typical restriction enzymes is illustrated in Fig. 2.

6

(3) Isolation of a mutant plasmid endowing a host microorganism with a high AHV resistance:

strain K53 carrying pChom1 was grown in NB medium containing 25 $\mu$g/m$\ell$ kanamycin up to the late stage of logarithmic growth phase. Cells were centrifuged twice with 50 mM Tris-maleic acid buffer solution (pH 6.0), suspended in 50 mM Tris-maleic acid buffer solution (pH 6.0) containing 400 $\mu$g/m$\ell$ N-methyl-N'-nitro-N-nitrosoguanidine, and treated at room temperature for 30 minutes. The treated cells were centrifuged and washed with the same buffer solution as used above twice. The cells were suspended in NB medium and subjected to shaking culture at 30°C for 2 hours. The cultured cells were centrifuged and washed twice with physiological saline solution, and suspended in physiological saline solution. The cell suspension was spread onto minimal agar medium M1 containing 20 $\mu$g/m$\ell$ kanamycin and 6 mg/m$\ell$ AHV, and cultured at 30°C for 3 days. One of the colonies formed was purified, and a plasmid was isolated from the cultured cells in the same manner as above. The plasmid was named pChom10.

Cultured cells of K53 strains containing pChom1 and pChom10, respectively, were centrifuged and washed with physiological saline solution, and the microorganisms in an amount corresponding to about $10^4$ cells were spread onto minimal agar media M1 containing 2 mg/m$\ell$, 4 mg/m$\ell$ and 6 mg/m$\ell$ AHV, respectively, to compare the degree of AHV resistance between these two strains. As a result of culturing at 30°C for 3 days, the strain carrying pChom1 grew on M1 agar medium containing 2 mg/m$\ell$ AHV, but failed to grow in the agar medium containing 4 mg/m$\ell$ AHV. On the other hand, the strain containing pChom10 grew even on M1 agar medium containing 6 mg/m$\ell$ AHV.

As a result of cleavage analysis with various restriction enzymes, it was found that pChom10 had the same structure as that of pChom1, and also had the ability to complement the HK-deficiency of the threonine-requiring mutant strain K54.

(4) Production of threonine by the strain containing pChom1 or pChom10:

Corynebacterium glutamicum ATCC 31833, Corynebacterium herculis ATCC 13868, Brevibacterium lactofermentum ATCC 13869 and a lysine-producing strain Brevibacterium flavum ATCC 21475 [resistant to S-(2-aminoethyl)cysteine] were transformed with pChom1 and pChom10. Protoplasts were prepared in the same manner as in the preparation of the protoplasts of strain K54 in step (2). That is, the protoplasts were prepared by treating the cells with penicillin G (0.45 unit/m$\ell$) in the course of culturing in SSM medium, and then by treating the cultured cells with lysozyme.

The protoplasts were transformed with 1 $\mu$g of the plasmid DNA in the same manner as above, and kanamycin-resistant transformants were selected on RCGP agar medium. The plasmids were isolated from the transformants in the same manner as in the isolation of the plasmid from the pChom1-transformed strain of strain K54 in step (2), and it was confirmed by cleavage analysis with various restriction enzymes that each transformant carried pChom1 or pChom10.

Threonine production tests on the transformants and their respective parent strains were carried out in the following manner.

First, 0.5 m$\ell$ of a seed culture obtained by shaking culture in NB medium at 30°C for 16 hours was inoculated in a test tube containing 5 m$\ell$ of a production medium [a medium containing 100g of glucose, 20g of $(NH_4)_2SO_4$, 0.5g of $KH_2PO_4$, 0.5g of $K_2HPO_4$, 1g of $MgSO_4 \cdot 7H_2O$, 10 mg of $FeSO_4 \cdot 7H_2O$, 10 mg of $MnSO_4 \cdot 4\text{-}6H_2O$, 100 $\mu$g of biotin, and 20g of calcium carbonate in 1$\ell$ of water, and adjusted to pH 7.2], and subjected to shaking culture at 30°C for 72 hours. After the culturing, the culture filtrate was subjected to paper chromatography, and the amount of the produced L-threonine was determined by colorimetry using ninhydrin coloration after paper chromatography. The results are shown in Table 1.

Table 1

| Strain | Amount of the threonine produced (g/ℓ) |
|---|---|
| Corynebacterium glutamicum ATCC31833 | 0 |
| " ATCC31833/pChom1 | 0.4 |
| " ATCC31833/pChom10 | 1.9 |
| Corynebacterium herculis ATCC13868 | 0 |
| " ATCC13868/pChom1 | 0.6 |
| " ATCC13868/pChom10 | 2.2 |
| Brevibacterium lactofermentum ATCC13869 | 0 |
| " ATCC13869/pChom1 | 0.3 |
| " ATCC13869/pChom10 | 1.7 |
| Brevibacterium flavum ATCC21475 | 0 |
| " ATCC21475/pChom1 | 0.4 |
| " ATCC21475/pChom10 | 5.2 |

(5) Production of isoleucine by the strain containing pChom1 or pChom10:

Corynebacterium glutamicum FERM P-7160 (FERM BP-455), Brevibacterium flavum ATCC 14067 and the lysine-producing strain Corynebacterium glutamicum FERM BP-158 were transformed in the same manner as in (4) above to obtain transformants carrying pChom1 and pChom10. It was confirmed in the same manner as above that the transformants had the plasmids. Isoleucine production tests on the parent strains and the transformants were carried out under the same conditions as in (4). The results are shown in Table 2.

## Table 2

| Strain | Amount of the isoleucine produced (g/ℓ) |
|---|---|
| Corynebacterium glutamicum FERM P-7160 | 1.2 |
| " FERM P-7160/pChom1 | 2.6 |
| " FERM P-7160/pChom10 | 5.3 |
| Brevibacterium flavum ATCC14067 | 0 |
| " ATCC14067/pChom1 | 0.6 |
| " ATCC14067/pChom10 | 3.7 |
| Corynebacterium glutamicum FERM BP-158 | 0 |
| " FERM BP-158/pChom1 | 0.9 |
| " FERM BP-158/pChom10 | 4.8 |

(6) Subcloning of HD and HK genes:

The recombinant plasmid containing the fragment between the SmaI cleavage site and the right cleavage site among the three PvuII cleavage sites illustrated in Fig. 2 as dark area was obtained in the following manner.

First, 3 units of EcoRI Takara Shuzo Co.) was added to 20 $\mu\ell$ of a reaction solution for EcoRI (100 mM Tris-HCl, 6 mM MgCl$_2$, and 50 mM NaCl, pH 7.5) containing one $\mu$g of pCE54 plasmid DNA. The reaction was carried out at 37°C for 60 minutes and stopped by heating at 70°C for 15 minutes. Deoxy ATP and deoxy TTP (0.05 mM each) were added, and then 3 units of Escherichia coli DNA polymerase I large fragment (Takara Shuzo Co.) was added. The reaction was carried out at 37°C for 30 minutes and stopped by heating at 70°C for 15 minutes.

Separately, 3 units of SmaI (Takara Shuzo Co.) and one unit of PvuII (Takara Shuzo Co.) were added to 20 $\mu\ell$ of a reaction solution for SmaI (10 mM Tris-HCl, 20 mM KCl, and 6 mM MgCl$_2$, pH 7.5) containing 3 $\mu$g of pChom1 plasmid DNA, and reaction was carried out at 37°C for 60 minutes. A DNA fragment of 2.6 Kb was obtained by fractionation and purification from the reaction product using the method described in Molecular Cloning (Cold Spring Harbor Laboratory, 1982) p.164. That is, the reaction product was subjected to agarose gel electrophoresis to cut off a gel band containing DNA of 2.6 Kb, and the gel band was subjected to electrophoresis in a dialysis membrane to extract the DNA fragment. A threefold volume of ethanol was added to the extract. The mixture was cooled at -80°C for 10 minutes and a precipitate was recovered by centrifugation. After evaporating the ethanol in vacuo, the residue was dissolved in 20 $\mu\ell$ of the reaction solution for EcoRI.

Both reaction products were mixed, and 5 $\mu\ell$ of a 10-fold concentrated buffer solution for T4 ligase 5 $\mu\ell$ of 5 mM ATP and one unit of T4 ligase (Takara Shuzo Co.) were added. Reaction was carried out at 12°C for 16 hours. Protoplasts of strain K53 were prepared by the method described above and transformation was carried out using the ligase reaction product. A plasmid DNA was prepared from one of the kanamycin-resistant and homoserine-non-requiring transformants by the method described above and was named pChom20.

pChom20 was cleaved with PstI and analyzed by agarose gel electrophoresis. As the result, it was confirmed that a region of 2.6 Kb containing a PstI fragment of 1.0 Kb of the inserted SalI fragment of 3.6 Kb of pChom1 and illustrated in Fig. 1 was subcloned in pChom20.

strain K53 and strain K54 were again transformed with pChom20. As a result of examination, it was confirmed that the plasmid DNA had the ability of complementing both HD and HK defects.

Activities of HD and HK were measured by the method described in J. Biochem. 68, 311 (1970) and ibid. 71, 219 (1972). As the result, the HD activity of the transformant of strain K54 carrying pChom20 was 16 times that of strain K54, and the HK activity of the transformant of strain K53 carrying pChom20 was 17 times that of strain K53. These results are the same as in the case of the transformants carrying pChom1. From the foregoing, it was confirmed that HD and HK genes were present on the fragment of 2.6 Kb from the SmaI restriction site to the PvuII restriction site.

Three units of HindIII (Takara Shuzo Co.) was added to 20 $\mu\ell$ of a reaction solution for HindIII (10 mM Tris-HCl, 6 mM MgCl$_2$, and 60 mM NaCl, pH 7.5) containing 1 $\mu$g of pChom1 DNA. Reaction was carried out at 37°C for 60 minutes and stopped by heating at 70°C for 15 minutes. Deoxy ATP, deoxy GTP, deoxy CTP and deoxy TTP (0.05 mM each) were added and 3 units of Escherichia coli DNA polymerase I large fragment (Takara Shuzo Co.) was added. Reaction was carried out at 37°C for 30 minutes and stopped by heating at 70°C for 15 minutes. One $\mu\ell$ of 2M NaCl was added and 3 units of SalI (Takara Shuzo Co.) was added. Reaction was carried out at 37°C for one hour. The reaction product was subjected to agarose gel electrophoresis. A DNA fragment of 2.5 Kb was purified by the method described above and dissolved in 20 $\mu\ell$ of the reaction solution for HindIII.

Separately, 3 units of EcoRI was added to 20 $\mu\ell$ of the reaction solution for EcoRI containing 1 $\mu$g of pCE54 plasmid DNA. The reaction was carried out at 37°C for 60 minutes and stopped by heating at 70°C for 15 minutes. Deoxy ATP and deoxy TTP (0.05 mM each) were added to the reaction product and further 3 units of Escherichia coli DNA polymerase I large fragment was added. The reaction was carried out at 37°C for 30 minutes and stopped by heating at 70°C for 15 minutes. One $\mu\ell$ of 2M NaCl was added to the reaction product and further 3 units of SalI was added. The reaction was carried out at 37°C for one hour and the reaction product was subjected to agarose gel electrophoresis. A fragment of 12.5 Kb was purified by the method described above and dissolved in 20 $\mu\ell$ of the reaction solution for HindIII.

Both solutions of DNA fragments were mixed, and 5 $\mu\ell$ of a 10-fold concentrated buffer solution for T4 ligase, 5 $\mu\ell$ of 5 mM ATP and 1 unit of T4 ligase were added. The reaction was carried out at 12°C for 16 hours. Protoplasts of strain K54 were prepared by the same method as mentioned above and transformation was carried out using the ligase reaction product. A plasmid DNA was prepared from one of the kanamycin-resistant and threonine-non-requiring transformants by the method mentioned above. It was confirmed by the analysis with restriction enzymes that the HindIII-SalI fragment of 2.5 Kb in the SalI fragment of 3.6 Kb was subcloned in the plasmid DNA named pChom21.

HD and HK activities of strain K54 carrying pChom20 or pChom21 were measured by the method described in J. Biochem. 68, 311 (1970) and ibid. 71, 219 (1972). As the result, it was found that the HD activity of the pChom21-carrying strain was less than 1/15 of that of the pChom20-carrying strain and the HK activity was more than 5/6. It was recognized from this fact that the region upstream from the HindIII cleavage site present in the region of 75 base pairs upstream from the initiation codon of HD is required for complete expression of HD.

(7) Nucleotide sequence of the DNA fragment containing both HD and HK genes:

The whole nucleotide sequence of the DNA fragment containing both HD and HK genes subcloned in pChom1 and pChom20 was determined using the method described in Methods in Enzymology 101, 20, 1983. That is, after a restriction cleavage map was prepared by a conventional method, a single stranded DNA was prepared by subcloning into the M13 phage vector and the nucleotide sequence was determined by the chain termination method using dideoxynucleotides

The result is illustrated in Table 3. Table 3 shows the nucleotide sequence of the SmaI-PvuII DNA fragment consisting of 2615 base pairs and the amino acid sequence corresponding to two open reading frames (from base No. 322 to base No. 1557 and from base No. 1571 to base No. 2497) present in the DNA fragment. These open reading frames correspond to the HD and HK structural genes, respectively.

ATCC 39019 strain containing pCE54 and ATCC 31833 strain have been deposited with the ATCC under the Budapest Treaty.

Table 3

```
                                                                  60
CCCGGGTTGATATTAGATTTCATAAATATACTAAAAATCTTGAGAGTTTTTCCGGTTGAA

                                                                 120
AACTAAAAAGCTGGGAAGGTGAATCGAATTTCGGGGCTTTAAAGCAAAAATGAACAGCTT

                                                                 180
GGTCTATAGTGGCTAGGTACCCTTTTTGTTTTGGACACATGTAGGGTGGCCGAAACAAAG

                                                                 240
TAATAGGACAACAACGCTCGACCGCGATTATTTTTGGAGAATCATGACCTCAGCATCTGC

                                                                 300
CCCAAGCTTTAACCCCGGCAAGGTCCCGGCTCAGCAGTCGGAATTGCCCTTTTAGGATTC

                                                                 360
GGAACAGTCGGCACTGAGGTGATGCGTCTGATGACCGAGTACGGTGATGAACTTGCGCAC
                            MetArgLeuMetThrGluTyrGlyAspGluLeuAlaHis

                                                                 420
CGCATTGGTGGCCCACTGGAGGTTCGTGGCATTGCTGTTTCTGATATCTCAAAGCCACGT
ArgIleGlyGlyProLeuGluValArgGlyIleAlaValSerAspIleSerLysProArg

                                                                 480
GAAGGCGTTGCACCTGAGCTGCTCACTGAGGACGCTTTTGCACTCATCGAGCGCGAGGAT
GluGlyValAlaProGluLeuLeuThrGluAspAlaPheAlaLeuIleGluArgGluAsp

                                                                 540
GTTGACATCGTCGTTGAGGTTATCGGCGGCATTGAGTACCCACGTGAGGTAGTTCTCGCA
ValAspIleValValGluValIleGlyGlyIleGluTyrProArgGluValValLeuAla

                                                                 600
GCTCTGAAGGCCGGCAAGTCTGTTGTTACCGCCAATAAGGCTCTTGTTGCAGATCACTCT
AlaLeuLysAlaGlyLysSerValValThrAlaAsnLysAlaLeuValAlaAspHisSer

                                                                 660
GCTGAGCTTGCTGATGCAGCGGAAGCCGCAAACGTTGACCTGTACTTCGAGGCTGCTGTT
AlaGluLeuAlaAspAlaAlaGluAlaAlaAsnValAspLeuTyrPheGluAlaAlaVal

                                                                 720
GCAGGCGCAATTCCAGTGGTTGGCCCACTGCGTCGCTCCCTGGCTGGCGATCAGATCCAG
AlaGlyAlaIleProValValGlyProLeuArgArgSerLeuAlaGlyAspGlnIleGln

                                                                 780
TCTGTGATGGGCATCGTTAACGGCACCACCAACTTCATCTTGGACGCCATGGATTCCACC
SerValMetGlyIleValAsnGlyThrThrAsnPheIleLeuAspAlaMetAspSerThr

                                                                 840
GGCGCTGACTATGCAGATTCTTTGGCTGAGGCAACTCGTTTGGGTTACGCCGAAGCTGAT
GlyAlaAspTyrAlaAspSerLeuAlaGluAlaThrArgLeuGlyTyrAlaGluAlaAsp

                                                                 900
CCAACTGCAGACGTCGAAGGCCATGACGCCGCATCCAAGGCTGCAATTTTGGCATCCATC
ProThrAlaAspValGluGlyHisAspAlaAlaSerLysAlaAlaIleLeuAlaSerIle
```

Table 3 (Cont'd)

```
                                                          960
GCTTTCCACACCCGTGTTACCGCGGATGATGTGTACTGCGAAGGTATCAGCAACATCAGC
AlaPheHisThrArgValThrAlaAspAspValTyrCysGluGlyIleSerAsnIleSer


                                                         1020
GCTGCCGACATTGAGGCAGCACAGCAGGCAGGCCACACCATCAAGTTGTTGGCCATCTGT
AlaAlaAspIleGluAlaAlaGlnGlnAlaGlyHisThrIleLysLeuLeuAlaIleCys


                                                         1080
GAGAAGTTCACCAACAAGGAAGGAAAGTCGGCTATTTCTGCTCGCGTGCACCCGACTCTA
GluLysPheThrAsnLysGluGlyLysSerAlaIleSerAlaArgValHisProThrLeu


                                                         1140
TTACCTGTGTCCCACCCACTGGCGTCGGTAAACAAGTCCTTTAATGCAATCTTTGTTGAA
LeuProValSerHisProLeuAlaSerValAsnLysSerPheAsnAlaIlePheValGlu


                                                         1200
GCAGAAGCAGCTGGTCGCCTGATGTTCTACGGAAACGGTTGCAGGTGGCGCGCAAACGGT
AlaGluAlaAlaGlyArgLeuMetPheTyrGlyAsnGlyCysArgTrpArgAlaAsnGly


                                                         1260
CTGCTGTGCTTGGCGACGTCGTTGGAGCCGCACGAAACAAGGTGCACGGTGGCCGCTGTC
LeuLeuCysLeuAlaThrSerLeuGluProHisGluThrArgCysThrValAlaAlaVal


                                                         1320
CAGGTGAGTCCACCTACGCTAACCTGCCGATCGCTGATTTCGGTGAGACCACCACTCGTT
GlnValSerProProThrLeuThrCysArgSerLeuIleSerValArgProProLeuVal


                                                         1380
ACCACCTCGACATGGATGTGGAAGATCGCGTGGGCGTTTTGGCTGAATTGGCTAGCCTGT
ThrThrSerThrTrpMetTrpLysIleAlaTrpAlaPheTrpLeuAsnTrpLeuAlaCys


                                                         1440
TCTCTGAGCAAGGAATCTCCCTGCGTAACAATCCGACAGGAAGAGCGCGATGATGATGCA
SerLeuSerLysGluSerProCysValThrIleArgGlnGluGluArgAspAspAspAla


                                                         1500
CGTCTGATCGTTGTCACCCACTCTGCGCTGGAATCTGATCTTTCCCGCACCGTTGAACTG
ArgLeuIleValValThrHisSerAlaLeuGluSerAspLeuSerArgThrValGluLeu


                                                         1560
CTGAAGGCTAAGCCTGTTGTTAAGGCAATCAACAGTGTGATCCGCCTCGAAAGGGACTAA
LeuLysAlaLysProValValLysAlaIleAsnSerValIleArgLeuGluArgAsp***


                                                         1620
T TTTACTGACATGGCAATTGAACTGAACGTCGGTCGTAAGGTTACCGTCACGGTACCTGGA
               MetAlaIleGluLeuAsnValGlyArgLysValThrValThrValProGly


                                                         1680
TCTTCTGCAAACCTCGGACCTGGCTTTGACACTTTAGGTTTGGCACTGTCGATATACGAC
SerSerAlaAsnLeuGlyProGlyPheAspThrLeuGlyLeuAlaLeuSerIleTyrAsp


                                                         1740
ACTGTCGAAGTGGAAATTATTCCATCTGGCTTGGAAGTGGAAGTTTTTGGCGAAGGCCAA
ThrValGluValGluIleIleProSerGlyLeuGluValGluValPheGlyGluGlyGln


                                                         1800
GGAGAAGTCCCTCTTGATGGCTCCCACCTGGTGGTTAAAGCTATTCGTGCTGGCCTGAAG
GlyGluValProLeuAspGlySerHisLeuValValLysAlaIleArgAlaGlyLeuLys
```

12

Table 3 (Cont'd)

```
                                                                    1860
GCAGCTGACGCTGAAGTGCCTGGATTGCGAGTGGTGTGCCACAACAACATTCCGCAGTCT
AlaAlaAspAlaGluValProGlyLeuArgValValCysHisAsnAsnIleProGlnSer


                                                                    1920
CGTGGTCTTGGTTCCTCTGCTGCAGCGGCGGTTGCTGGTGTTGCAGCAGCTAATGGTTTG
ArgGlyLeuGlySerSerAlaAlaAlaAlaValAlaGlyValAlaAlaAlaAsnGlyLeu


                                                                    1980
GCGGATTTTCCGCTGACTCAAGAGCAGATTGTTCAGTTGTCCTCTGCCTTTGAAGGCCAC
AlaAspPheProLeuThrGlnGluGlnIleValGlnLeuSerSerAlaPheGluGlyHis


                                                                    2040
CCAGATAATGCTGCGGCTTCTGTGCTGGGCGGACGAGTGGTGTCGTGGACAAATCTGTCT
ProAspAsnAlaAlaAlaSerValLeuGlyGlyArgValValSerTrpThrAsnLeuSer


                                                                    2100
ATCGACGGCAAGAGCCAGCCACAGTATGCTGCTGTACCACTTGAGGTGCAGGATAATATT
IleAspGlyLysSerGlnProGlnTyrAlaAlaValProLeuGluValGlnAspAsnIle


                                                                    2160
CGTGCGACTGCGCTGGTTCCTAATT.TCCACGCATCCACCGAAGCTGTGCGCCGAGTCCTT
ArgAlaThrAlaLeuValProAsnPheHisAlaSerThrGluAlaValArgArgValLeu


                                                                    2220
CCAACTGAAGTCACTCACATCGATGCGCGATTCAACGTGTCCCGCGTTGCGGTGATGATC
ProThrGluValThrHisIleAspAlaArgPheAsnValSerArgValAlaValMetIle


                                                                    2280
GTTGCATTGCAGCAGCGTCCTGATCTGCTGTGGGAGGGTACTCGTGACCGACTGCACCAG
ValAlaLeuGlnGlnArgProAspLeuLeuTrpGluGlyThrArgAspArgLeuHisGln


                                                                    2340
CCTTATCGTGCAGAAGTGTTGCCCGTTACCTCCGAATGGGTAAACCGTCTGCGCAACCGT
ProTyrArgAlaGluValLeuProValThrSerGluTrpValAsnArgLeuArgAsnArg


                                                                    2400
GGCTATGCAGCGTACCTTTCCGGTGCCGGCCCAACCGCCATGGTGCTGTCCACTGAGCCA
GlyTyrAlaAlaTyrLeuSerGlyAlaGlyProThrAlaMetValLeuSerThrGluPro


                                                                    2460
ATTCCAGACAAGGTTTTGGAAGATGCTCGTGAGTCTGGCATTAAGGTGCTTGAGCTTGAG
IleProAspLysValLeuGluAspAlaArgGluSerGlyIleLysValLeuGluLeuGlu


                                                                    2520
GTTGCGGGACCAGTCAAGGTTGAAGTTAACCAACCTTAGGCCCAACAAGGAAGCCCCCTT
ValAlaGlyProValLysValGluValAsnGlnPro***


                                                                    2580
CGAATCAAGAAGGGGGCCTTATTAGTGAGCAATTATTCGCTGAACACGTGAACCTTACA


GGTGCCCGGCGCGTTGAGTGGTTTAGTTCCAGCTG
```

As illustrated in Table 4, similar base sequences exist in the region of 120 base pairs upstream from the initiation codon of the open reading frame of the HD gene and that upstream from the initiation codon of the open reading frame of the HK gene (the C-terminal region of the open reading frame of the HD gene). These sequences are required for the expression of both genes. Further, as indicated with the underline in Table 5, a potential stem loop structure is detected within 60 base pairs downstream from the termination codon of the open reading frame of the HK gene, and is considered as the transcription termination signal of the bacteria belonging to the genus Corynebacterium or Brevibacterium.

In Table 4, the similar sequences of the HD and HK genes are illustrated. The underlined parts are common in both sequences. The amino acid sequence from the initiation codon to the fifth residue is illustrated under the DNA sequences. "Stp" upstream from the HK initiation codon shows the termination codon of the HD gene.

Table 5 shows the DNA sequence of the C-terminal region of the HK gene and the region downstream therefrom.

**Claims**

Table 4

Hind III
↓

HD : CGATTATTTTTGGAGAATCATGACCTCAGCATCTGCCCCAAGCTTTAA

CCCCGGCAAGGTCCCGGC

HK : TCACCCACTCTGCGCTGGAATCTGATCTTTCCCGCACCGTTGAACTGC

TGAAGGCTAAGCCTGTTG


TCAGCAGTCGGAATTGCCCTTTTAGGATTCGGAACAGTCGGCACTGAG

GTGATGCGTCTGATGACC
   MetArgLeuMetThr·

TTAAGGCAATCAACAGTGTGATCCGCCTCGAAAGGGACTAATTTTACTGA
                                    Stp

CATGGCAATTGAACTG
MetAlaIleGluLeu


Table 5


GTTAACCAACCTTAGGCCCAACAAGGAAGCCCCCTTCGAATCAAGAAGGGGGCCTT
ValAsnGlnProStp

ATTAGTGAGCAA


1. A process for producing L-threonine or L-isoleucine, which comprises culturing in a medium a microorganism belonging to the genus Corynebacterium or Brevibacterium and containing a recombinant DNA of a DNA fragment carrying genetic information responsible for the synthesis of homoserine dehydrogenase and homoserine kinase of a microorganism belonging to the genus Corynebacterium or Brevibacterium and of a vector DNA, accumulating L-threonine or L-isoleucine in the culture broth, and recovering L-threonine or L-isoleucine therefrom.

2. A recombinant DNA which contains a DNA fragment carrying genetic information responsible for the synthesis of homoserine dehydrogenase and homoserine kinase originating from a microorganism belonging to the genus Corynebacterium or Brevibacterium and capable of endowing a host microorganism belonging to the genus Corynebacterium or Brevibacterium with a resistance to an analog of threonine or isoleucine.

3. A microorganism belonging to the genus Corynebacterium or Brevibacterium and containing a recombinant DNA of a DNA fragment carrying genetic information responsible for the synthesis or homoserine dehydrogenase and homoserine kinase originating from a microorganism belonging to the genus Corynebacterium or Brevibacterium and of a vector DNA.

4. A DNA coding for the amino acid sequence of homoserine dehydrogenase as illustrated in Table 3.

5. A DNA coding for the amino acid sequence of homoserine kinase as illustrated in Table 3.

6. A process for producing L-threonine or L-isoleucine, which comprises introducing a recombinant DNA containing a DNA fragment carrying genetic information responsible for the synthesis of homoserine dehydrogenase and homoserine kinase of a microorganism belonging to the genus Corynebacterium or Brevibacterium into a lysine-producing microorganism belonging to the genus Corynebacterium or Brevibacterium, culturing the thus obtained transformant in a medium, accumulating L-threonine or L-isoleucine in the medium and recovering L-threonine or L-isoleucine therefrom.

7. A DNA sequence necessary for expressing a foreign gene in a microorganism belonging to the genus Corynebacterium or Brevibacterium, which contains 120 base pairs upstream from the initiation codon of the open reading frame coding for homoserine dehydrogenase or a part thereof and 60 base pairs downstream from the termination codon of the open reading frame coding for homoserine kinase or a part thereof as shown in the DNA sequence of Table 3.

## Revendications

1. Procédé de production de L-thréonine ou de L-isoleucine, qui comprend la culture, dans un milieu, d'un microorganisme appartenant au genre Corynebacterium ou Brevibacterium et contenant un ADN recombinant d'un fragment d'ADN, portant l'information génétique responsable de la synthèse de l'homosérine déhydrogénase et de l'homosérine kinase d'un microorganisme appartenant au genre Corynebacterium ou Brevibacterium, et d'un ADN vecteur, l'accumulation de la L-thréonine ou de la L-isoleucine dans le bouillon de culture, et la récupération de la L-thréonine ou de la L-isoleucine à partir de celui-ci.

2. Un ADN recombinant qui contient un fragment d'ADN portant l'information génétique responsable de la synthèse de l'homosérine déhydrogénase et de l'homosérine kinase provenant d'un microorganisme appartenant au genre Corynebacterium ou Brevibacterium, et capable de doter un microorganisme hôte appartenant au genre Corynebacterium ou Brevibacterium d'une résistance à un analogue de la thréonine ou de l'isoleucine.

3. Microorganisme appartenant au genre Corynebacterium ou Brevibacterium et contenant un ADN recombinant d'un fragment d'ADN, portant l'information génétique responsable de la synthèse de l'homosérine déhydrogénase et de l'homosérine kinase provenant d'un microorganisme appartenant au genre Corynebacterium ou Brevibacterium, et d'un ADN vecteur.

4. Un ADN codant pour la séquence d'acides aminés de l'homosérine déhydrogénase, tel qu'illustré dans le Tableau 3.

5. Un ADN codant pour la séquence d'acides aminés de l'homosérine kinase, tel qu'illustré dans le Tableau 3.

6. Procédé de production de la L-thréonine ou de la L-isoleucine, qui comprend l'introduction d'un ADN recombinant, qui contient un fragment d'ADN portant l'information génétique responsable de la synthèse de l'homosérine déhydrogénase et de l'homosérine kinase d'un microorganisme appartenant au genre Corynebacterium ou Brevibacterium, dans un microorganisme producteur de lysine et appartenant au genre Corynebacterium ou Brevibacterium, la culture du transformant ainsi obtenu dans un milieu, l'accumulation de la L-thréonine ou de la L-isoleucine dans le milieu, et la récupération de la L-thréonine ou de la L-isoleucine à partir de celui-ci.

7. Séquence d'ADN nécessaire pour l'expression d'un gène étranger dans un microorganisme appartenant au genre Corynebacterium ou Brevibacterium, qui contient 120 paires de bases situées en amont du codon d'initiation du cadre ouvert de lecture codant pour l'homosérine déhydrogénase, ou une partie de celles-ci, et 60 paires de bases situées en aval du codon de terminaison du cadre ouvert de lecture codant pour l'homosérine kinase, ou une partie de celles-ci, dans la séquence d'ADN du Tableau 3.

**Patentansprüche**

1. Verfahren zur Herstellung von L-Threonin oder L-Isoleucin, umfassend die Züchtung eines Mikroorganismus der Gattung Corynebacterium oder Brevibacterium, in einem Medium, wobei der Mikroorganismus eine rekombinante DNA aus einem DNA-Fragment, welches die genetische Information trägt, die für die Synthese von Homoserin-Dehydrogenase und Homoserin-Kinase eines Mikroorganismus der Gattung Corynebacterium oder Brevibacterium verantwortlich ist, und einer Vektor-DNA umfaßt, Anreicherung von L-Threonin oder L-Isoleucin in der Kulturbrühe, und Gewinnung von L-Threonin oder L-Isoleucin daraus.

2. Rekombinante DNA, enthaltend ein DNA-Fragment, das die genetische Information trägt, welche für die Synthese von Homoserin-Dehydrogenase und Homoserin-Kinase verantwortlich ist, die von einem Mikroorganismus der Gattung Corynebacterium oder Brevibacterium stammen, und die fähig ist, einem Wirtsmikroorganismus der Gattung Corynebacterium oder Brevibacterium Resistenz gegenüber einem Analog von Threonin oder Isoleucin zu verleihen.

3. Mikroorganismus der Gattung Corynebacterium oder Brevibacterium, enthaltend eine rekombinante DNA aus einem DNA-Fragment, welches die genetische Information trägt, die für die Synthese von Homoserin-Dehydrogenase und Homoserin-Kinase verantwortlich ist. welche aus einem Mikroorganismus der Gattung Corynebacterium oder Brevibacterium stammen, und einer Vektor-DNA.

4. DNA, codierend für die Aminosäuresequenz von Homoserin-Dehydrogenase gemäß der Beschreibung in Tabelle 3.

5. DNA, codierend für die Aminosäuresequenz von Homoserinkinase gemäß der Beschreibung in Tabelle 3.

6. Verfahren zur Herstellung von L-Threonin oder L-Isoleucin, umfassend die Einführung einer rekombinanten DNA, die ein DNA-Fragment enthält, welches die genetische Information trägt, die für die Synthese von Homoserin-Dehydrogenase und Homoserin-Kinase eines Mikroorganismus der Gattung Corynebacterium oder Brevibacterium verantwortlich ist, in einen Lysin-produzierenden Mikroorganismus der Gattung Corynebacterium oder Brevibacterium, die Züchtung des so erhaltenen Transformanten in einem Medium, die Anreicherung von L-Threonin oder L-Isoleucin in dem Medium und die Gewinnung von L-Threonin oder L-Isoleucin daraus.

7. DNA-Sequenz, die für die Expression eines fremden Gens in einem Mikroorganismus der Gattung Corynebacterium oder Brevibacterium erforderlich ist, enthaltend 120 Basenpaare stromaufwärts vom Initiationscodon des offenen Leserahmens, die für die Homoserin-Dehydrogenase oder einen Teil davon codieren, und 60 Basenpaare stromabwärts vom Terminationscodon des offenen Leserahmens, die für die Homoserin-Kinase oder einen Teil davon codieren, wie in der DNA-Sequenz von Tabelle 3 gezeigt.

Fig. 1

Fig. 2